(19) Europäisches Patentamt
European Patent Office
Office européen des brevets

(11) EP 2 522 341 A1

(12) EUROPEAN PATENT APPLICATION

(43) Date of publication:
**14.11.2012 Bulletin 2012/46**

(51) Int Cl.:
***A61K 31/17*** *(2006.01)* ***A61P 35/00*** *(2006.01)*

(21) Application number: **11166117.9**

(22) Date of filing: **13.05.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Tragex Pharma**
**75015 Paris (FR)**

(72) Inventors:
- **Hadj-Slimane, Reda**
**75015 Paris (FR)**
- **Garbay, Christiane**
**75015 Paris (FR)**

(74) Representative: **Icosa**
**83 avenue Denfert-Rochereau**
**75014 Paris (FR)**

# (54) Pharmaceutical compositions comprising Neuropilin inhibitors, and their use for the prevention and/or treatment of angiogenic disorders and cancers

(57) The present invention relates to a pharmaceutical composition comprising a compound of general formula (I),

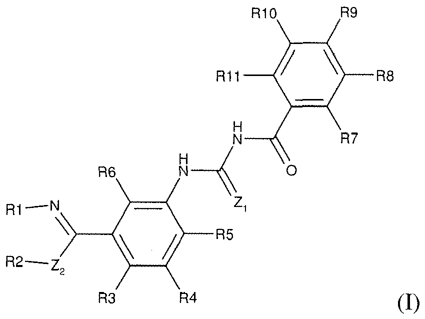

(I)

wherein
- **Z1** is S or O, preferably S;
- -**Z2**- is -N**R^a^**-, wherein $\mathbf{R^a}$ is H, OH, alkyl, alkoxy, aryle, alkenyl, alkynyl, amine, preferably $R^a$ is H; -N=; O; S; -C$\mathbf{R^b}$=, wherein $\mathbf{R^b}$ is H, OH, alkyl, alkoxy, aryle, alkenyl, alkynyl, amine, $NO_2$, Cl, Br, I, F, preferably H; -CH$\mathbf{R^c}$-, wherein $\mathbf{R^c}$ is H, OH, alkyl, alkoxy, aryle, alkenyl, alkynyl, amine, $NO_2$, Cl, Br, I, F, or -$CH_2$-; preferably -**Z2**- is -NH- or -N=;
- each of **R1** and **R2** is independently H, OH, alkyl, alkoxy, aryle, alkenyl, alkynyl, amine, $NO_2$, Cl, Br, I, F; preferably **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;
- **R5** is H, OH, $C_{1-4}$ alkyl, $C_{1-4}$ alkoxy, $C_{1-4}$ alkenyl, $C_{1-4}$ alkynyl, amine, preferably **R5** is $NH_2$, $NO_2$, Cl, F, Br, I; more preferably **R5** is H or $CH_3$; even more preferably **R5** is $CH_3$;
- each of **R8** and **R9** is independently, H, OH, alkyl, alkoxy, aryle, alkenyl, alkynyl, amine, $NO_2$, Cl, Br, I, F; preferably $R_8$ and $R_9$ together form a cycle comprising 5 or 6 atoms, preferably an heterocycle comprising 5 or 6 atoms, more preferably a 1,3-dioxacyclopentene or a 1,4-dioxane; and
- each of **R3, R4, R6, R7, R10** and **R11** is independently, H, OH, $C_{1-4}$ alkyl, $C_{1-4}$ alkoxy, $C_{1-4}$ alkenyl, $C_{1-4}$ alkynyl, amine, $NO_2$, F, Cl, Br, I; preferably H, $CH_3$, $OCH_3$, OH, $NH_2$, $NO_2$, Cl, F, Br, I; more preferably is H or $CH_3$; even more preferably is H,

or esters or salts thereof; in association with at least one pharmaceutically acceptable vehicle; and to the use thereof for inhibiting the Neuropilin pathways in the treatment of cancer and of angiogenic diseases.



Printed by Jouve, 75001 PARIS (FR)

## Description

### FIELD OF INVENTION

The present invention relates to the field of the treatment and/or prevention of diseases, disorders or conditions related to cancers and/or angiogenesis. The present invention particularly relates to specific chemical compounds, herein shown to be inhibitors of the Neuropilin pathways, and to the pharmaceutical use thereof, especially for treating and/or preventing diseases, disorders or conditions related to cancers and/or angiogenesis.

### BACKGROUND OF INVENTION

Neuropilin is a transmembrane glycoprotein acting as a co-receptor for two types of ligands: members of the semaphorin family, a family of axon guidance modulators, and members of the vascular endothelial growth factor (VEGF) family. Neuropilin exists in two forms, namely Neuropilin - 1 (NRP-1) and 2 (NRP-2). NRP-1 and NRP-2 present different affinity and specificity of ligands. Both proteins may function as co-receptors binding an extracellular ligand with high affinity, and complexing with other transmembrane receptors of the same ligand (e.g. VEGF-R2 for VEGF), to modulate signal transduction within the cell.

The overexpression of NRP-1 and NRP-2 has been demonstrated to be correlated with tumor progression and patient prognosis in specific tumor types. Moreover, Neuropilin seems to contribute to tumor formation in a number of diseases such as prostate, breast and colon cancer (Ellis LM, Mol Cancer Ther, 2006, 5(5): 1099-107). Neuropilin may mediate the effects of VEGF and semaphorins on the proliferation, survival and migration of cancer cells.

Neuropilin may thus be a promising target for preventing and/or treating diseases, disorders or conditions related to cancer.

Moreover, Neuropilin is involved, as a member of the VEGF pathway, in the process of angiogenesis. Angiogenesis is a fundamental process of new blood vessels formation by endothelial cells. This formation involves three different steps, (i) the migration, (ii) the growth and (iii) the differentiation of endothelial cells. Disturbance of this tightly regulated phenomenon may lead to several diseases, wherein tissues and organs are subjected to the invasion by neovessels.

Therefore, an inhibitor of Neuropilin may also be useful for the prevention and/or the treatment of angiogenic diseases, disorders or conditions.

Several means for inhibiting Neuropilin have been described in the art. For example, WO03/035100 describes a modulator of the expression or activity of Neuropilin. WO2007/056470 describes antibodies against NRP-1. US7,335,357 describes proteins or protein fragments, especially members of the semaphorin collapsin family, as inhibitors of Neuropilin. WO2009/099959 and WO99/55855 describe nucleic acids, such as, for example, antisens oligonucleotides, for modulating the expression of Neuropilin genes. Jarvis et al. (J. Med. Chem. 2010, 53, 2215-26) describes particular chemical compounds (such as, for example, EG00229) for inhibiting the interaction between NRP-1 and VEGF-A.

However, to the Applicant knowledge, there are no clinical trials or current medical development susceptible to lead to a medicament, involving a small molecule inhibiting Neuropilin.

Therefore, due to the great potential of such compounds for preventing and/or treating diseases, disorders or conditions related to cancers and/or angiogenesis, there is still a need for a small molecule, such as, for example, a chemical compound, for inhibiting the neuropilin pathway.

This invention relates to pharmaceutical compositions comprising chemical compounds of general formula (I),

(I)

wherein

- **Z1** is S or O, preferably S;
- -**Z2**- is -N**R^a^**-, wherein **R^a^** is H, OH, alkyl, alkoxy, aryle, alkenyl, alkynyl, amine, preferably **R^a^** is H; -N=; -O-; -S-; -C**R^b^**=, wherein **R^b^** is H, OH, alkyl, alkoxy, aryle, alkenyl, alkynyl, amine, $NO_2$, Cl, Br, I, F, preferably H; -CH**R^c^**-, wherein **R^c^** is H, OH, alkyl, alkoxy, aryle, alkenyl, alkynyl, amine, $NO_2$, Cl, Br, I, F, or -$CH_2$-; preferably -**Z2**- is -NH- or -N=;
- each of **R1** and **R2** is independently H, OH, alkyl, alkoxy, aryle, alkenyl, alkynyl, amine, $NO_2$, Cl, Br, I, F; preferably **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;
- **R5** is H, OH, $C_{1-4}$ alkyl, $C_{1-4}$ alkoxy, $C_{1-4}$ alkenyl, $C_{1-4}$ alkynyl, amine, $NO_2$, Cl, F, Br, I; more preferably **R5** is H or $CH_3$; even more preferably **R5** is $CH_3$;
- each of **R8** and **R9** is independently, H, OH, alkyl, alkoxy, aryle, alkenyl, alkynyl, amine, $NO_2$, Cl, Br, I, F; preferably **R8** and **R9** together form a cycle comprising 5 or 6 atoms, preferably an heterocycle comprising 5 or 6 atoms, more preferably a 1,3-dioxacyclopentene or a 1,4-dioxane; and
- each of **R3, R4, R6, R7, R10** and **R11** is independently, H, OH, $C_{1-4}$ alkyl, $C_{1-4}$ alkoxy, $C_{1-4}$ alkenyl, $C_{1-4}$ alkynyl, amine, $NO_2$, F, Cl, Br, I; preferably H, $CH_3$, $OCH_3$, OH, $NH_2$, $NO_2$, Cl, F, Br, I; more preferably is H or $CH_3$; even more preferably is H,

or esters or salts thereof; in association with at least one pharmaceutically acceptable vehicle.

After having found the compounds of the invention and their interest as neuropilin inhibitors, the Applicant got aware of W02009/130422 relating to chemical compounds for inhibiting the Hedgehog pathways. The experiments of WO2009/130422 are carried out exclusively on embryonic cell lines. The results of these experiments showed that the chemical compounds of W02009/130422 inhibited the differentiation of embryonic cell lines (embryonic mouse cells of the adipocytes lineage C3H10T1/2 and human embryonic kidney cells, HEK293).

WO2009/130422 does not give any teaching on the NRP pathways. Even though Neuropilin may be involved in embryonic development, it is generally recognized that Neuropilin impacts processes of embryonic endothelial cells or nerve cells only (vessel formation and nerve fiber guidance). And W02009/130422 gives information on adipocytes and kidney cell lines differentiation exclusively.

Moreover, the effect of the compounds of WO2009/130422 on the differentiation of embryonic cells, which are not cancer cells or endothelial cells, does not provide any evidence that these compounds may be useful in preventing and/or treating diseases, disorders or conditions related to cancers and/or angiogenesis.

The Inventors herein show that the chemical compounds of the invention inhibit the interaction between Neuropilin and the components of the VEGF pathway. Without willing to be bound to a theory, the Inventors suggest that the chemical compounds of the invention physically interact with the Neuropilin molecule, and thus block the associated signaling pathways, thereby providing beneficial effect for preventing and/or treating diseases associated to the neuropilin

pathways, such as, for example, cancers (e.g. solid tumor or metastasis) or diseases associated with abnormal angiogenesis (e.g. atherosclerosis, proliferative retinopathies, or rheumatoid arthritis).

## DEFINITIONS

"**Subject**": an animal, including a human. In the sense of the present invention, a subject may be a patient, i.e. a person receiving medical attention, undergoing or having underwent a medical treatment, or monitored for the development of a disease.

"**Treating a disease**": refers to reducing or alleviating at least one adverse effect or symptom of a disease, disorder or condition associated with a deficiency in an organ, tissue or cell function.

"**Preventing a disease**": refers to preventing or avoiding the occurrence of symptom. In the present invention, the term "prevention" may refer to a secondary prevention, i.e. to the prevention of the re-occurrence of a symptom or a relapse of the disease. It may also refer, when the disease is cancer, to the occurrence of metastases after the treatment and/or the removal of a tumor.

"**Therapeutically effective amount**": refers to the amount of a therapeutic agent necessary and sufficient for preventing a disease; slowing down or stopping the progression, aggravation, or deterioration of one or more symptoms of the disease or condition; alleviating the symptoms of the disease or condition; curing or treating the disease or condition.

"**Alkyl**": means any saturated linear or branched hydrocarbon chain, with 1 to 12 carbon atoms, preferably 1 to 6 carbon atoms, and more preferably methyl, ethyl, propyl, isopropyl, n-butyl, sec-butyl, isobutyl and tert-butyl.

"**Alkenyl**": means any linear or branched hydrocarbon chain having at least one double bond, of 2 to 12 carbon atoms, and preferably 2 to 6 carbon atoms.

"**Alkynyl**": means any linear or branched hydrocarbon chain having at least one triple bond, of 2 to 12 carbon atoms, and preferably 2 to 6 carbon atoms.

"**Amine**": means any compound derived from ammoniac $NH_3$ by substitution of one or more hydrogen atoms with an organic radical, preferably in the invention, the term amine stands for $NH_2$ or NH**R**, wherein **R** is an alkyl, preferably a $C_{1-4}$alkyl.

"**Aryle**": means a mono- or polycyclic system of 5 to 20, and preferably 6 to 12, carbon atoms having one or more aromatic rings (when there are two rings, it is called a biaryl) among which it is possible to cite the phenyl group, the biphenyl group, the 1-naphthyl group, the 2-naphthyl group, the tetrahydronaphthyl group, the indanyl group and the binaphthyl group. The term aryl also means any aromatic ring including at least one heteroatom chosen from an oxygen, nitrogen or sulfur atom. The aryl group can be substituted by 1 to 3 substituents chosen independently of one another, among a hydroxyl group, a linear or branched alkyl group comprising 1, 2, 3, 4, 5 or 6 carbon atoms, in particular methyl, ethyl, propyl, butyl, an alkoxy group or a halogen atom, in particular bromine, chlorine and iodine.

"**Alkoxy**": refers to any O-alkyl or O-aryl group.

"**Heterocycle**": means a ring with 5 or 6 links containing 1 to 2 heteroatoms chosen from O, S, N, optionally substituted with an alkyl.

## DETAILED DESCRIPTION

### [Pharmaceutical composition]

The present invention relates to a pharmaceutical composition comprising a compound of general formula (I):

(I)

wherein :

- **Z1** is S or O, preferably S;
- -**Z2**- is -N**R^a**-, wherein **R^a** is H, OH, alkyl, alkoxy, aryle, alkenyl, alkynyl, amine, preferably H; -N=; -O-; -S-; -C**R^b**=, wherein **R^b** is H, OH, alkyl, alkoxy, aryle, alkenyl, alkynyl, amine, $NO_2$, Cl, Br, I, F, preferably H; -CH**R^c**-, wherein **R^c** is H, OH, alkyl, alkoxy, aryle, alkenyl, alkynyl, amine, $NO_2$, Cl, Br, I, F, or -$CH_2$-, preferably -**Z2**- is -NH- or -N=;
- each of **R1** and **R2** is independently H, OH, alkyl, alkoxy, aryle, alkenyl, alkynyl, amine, $NO_2$, Cl, Br, I, F; preferably **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;
- **R5** is H, OH, $C_{1-4}$ alkyl, $C_{1-4}$ alkoxy, $C_{1-4}$ alkenyl, $C_{1-4}$ alkynyl, amine, $NO_2$, F, Cl, Br, I; preferably **R5** is H, $CH_3$, $OCH_3$, OH, $NH_2$, $NO_2$, Cl, F, Br, I; more preferably **R5** is H or $CH_3$; even more preferably **R5** is $CH_3$;
- each of **R8** and **R9** is independently H, OH, alkyl, alkoxy, aryle, alkenyl, alkynyl, amine, $NO_2$, Cl, Br, I, F; preferably **R8** and **R9** together form a cycle comprising 5 or 6 atoms, preferably an heterocycle comprising 5 or 6 atoms, more preferably a 1,3-dioxacyclopentene or a 1,4-dioxane; and
- each of **R3, R4, R6, R7, R10** and **R11** is independently H, OH, $C_{1-4}$ alkyl, $C_{1-4}$ alkoxy, $C_{1-4}$ alkenyl, $C_{1-4}$ alkynyl, amine, preferably $NH_2$, $NO_2$, F, Cl, Br, I; preferably H, $CH_3$, $OCH_3$, OH, $NH_2$, $NO_2$, Cl, F, Br, I; more preferably is H or $CH_3$; even more preferably is H;

or esters or salts thereof; in association with at least one pharmaceutically acceptable vehicle.

According to one embodiment, the compound of general formula (I) of the invention is of general formula (I) wherein

- **Z1** is S or O, preferably S;
- -**Z2**- is -N**R^a**-, wherein **R^a** is H, OH, alkyl, alkoxy, aryle, alkenyl, alkynyl, amine, preferably H; -N=; -O-; -S-; -C**R^b**=, wherein **R^b** is H, OH, alkyl, alkoxy, aryle, alkenyl, alkynyl, amine, $NO_2$, Cl, Br, I, F, preferably H; -CH**R^c**-, wherein **R^c** is H, OH, alkyl, alkoxy, aryle, alkenyl, alkynyl, amine, $NO_2$, Cl, Br, I, F, or -$CH_2$-, preferably -**Z2**- is -NH- or -N=;
- **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;
- **R5** is H or $C_{1-4}$ alkyl;
- **R8** and **R9** together form a cycle comprising 5 or 6 atoms, preferably an heterocycle comprising 5 or 6 atoms, more preferably a 1,3-dioxacyclopentene or a 1,4-dioxane;
- **R3, R4, R6, R7, R10** and **R11** are H or $C_{1-4}$alkyl.

According to one embodiment, the compound of general formula (I) of the invention is of general formula (I)

wherein

- **Z1** is S;
- -**Z2**- is -NH- or -N= or -S-;
- **R1** and **R2** together and with N and Z2 form a substituted imidazole and more preferably a benzimidazole;
- **R5** is H or $CH_3$ or $C_2H_5$;
- **R8** and **R9** together form a cycle comprising 5 or 6 atoms, preferably an heterocycle comprising 5 or 6 atoms, more preferably a 1,3-dioxacyclopentene or a 1,4-dioxane;
- **R3, R4, R6, R7, R10** and **R11** are H.

According to one embodiment, the compound of formula (I) of the invention has the general formula (Ia):

(Ia)

wherein

- **R5** is H or $CH_3$;
- **R8** and **R9** together form a 1,3-dioxacyclopentene or a 1,4-dioxane.

Preferably, the compound of formula (I) of the invention has the general formula (Ia) wherein

- **R5** is H or $CH_3$;
- **R8** and **R9** together form a 1,4-dioxane.

Preferred compounds of formula (I) include:

- N-[5-(1H-benzimidazol-2-yl)-2-methylphenyl]-N'-(2,3-dihydro-1,4-benzodioxin -6-ylcarbonyl)thiourea (compound **1**)

- N-[3-(1H-benzimidazol-2-yl)phenyl]-N'-(2,3-dihydro-1,4-benzodioxin -6- ylcarbonyl)thiourea (compound **2**) :

- N-[3-(1H-benzimidazol-2-yl)phenyl]-N'-(1,3-benzodioxol-5-ylcarbonyl)thiourea (compound **3**)

Below are some of the chemical characteristics of the compounds **1** and **2** of the invention.

| | Compound 1 | Compound 2 |
|---|---|---|
| Molecular formula | $C_{24}H_{20}N_4O_3S$ | $C_{23}H_{17}N_4O_3S$ |
| State | Powder | Powder |
| Colour | Yellow | Yellow |
| Molecular weight <500 | 444 g/mol | 430 g/mol |
| H-bond donors <5 | 4 | 3 |
| H-bond acceptor <10 | 3 | 5 |
| logP <5 | 3.15 | 2.94 |
| Melting point | 202 | 212 |

In one embodiment, the pharmaceutical composition of the invention comprises several compounds of general formula (I) or esters or salts thereof. In one embodiment, the composition of the invention comprises the compound **1** and the compound **2** as hereinabove described. In one embodiment, the composition of the invention comprises the compound **1** and the compound **3** as hereinabove described. In one embodiment, the composition of the invention comprises the compound **2** and the compound **3** as hereinabove described. In one embodiment, the composition of the invention comprises the compound **1,** the compound **2** and the compound **3** as hereinabove described.

The present invention also relates to a medicament comprising the pharmaceutical composition as hereinabove described.

This invention also relates to a method for manufacturing compositions as hereinabove described, wherein a compound of general formula (II)

(II)

wherein **R1, R2, Z2, R3, R4, R5** and **R6** are as described hereinabove,
is reacted with a compound of general formula (IV)

(IV)

in a refluxing solvent, such as, for example, acetone, to give compounds of general formula (I).

According to an embodiment, the compound of general formula (IV) is obtained by reacting a compound of general formula (III)

(III)

with (i) $SOCl_2$ and (ii) $NH_4$**Z1**CN, wherein **Z1** is as hereinabove defined, in the presence of a base, such as, for example, $K_2CO_3$ in a refluxing solvent, such as, for example, acetone.

The reaction process is shown below.

1) $SOCl_2$

2) $NH_4$**Z1**CN

(III) (IV)

(II) + (IV) → (I)

The reaction process for synthetizing the compound **1** of the invention is shown below:

*Compound 1*

The invention also relates to a pharmaceutical composition as hereinabove described for inhibiting the Neuropilin pathways.

The invention also relates to a method for inhibiting the Neuropilin pathways in a subject in need thereof, comprising administering to said subject a therapeutically effective amount of a pharmaceutical composition comprising a compound of general formula (I).

The invention also relates to an inhibitor of the Neuropilin pathways, which is a pharmaceutical composition or a compound of general formula (I) as hereinabove described. Surprisingly, the Inventors showed that the compounds of the invention inhibited the VEGF - NRP-1 binding (see Examples). Without willing to be bound to a theory, the Inventors suggest that the chemical compounds of the invention physically interact with the Neuropilin molecule, and thus block the associated signaling pathways

The present invention also relates to a pharmaceutical composition comprising a therapeutically effective amount of a compound of general formula (I) in association with a pharmaceutically acceptable vehicle, for use in the prevention and/or the treatment of a cancer in a subject in need thereof.

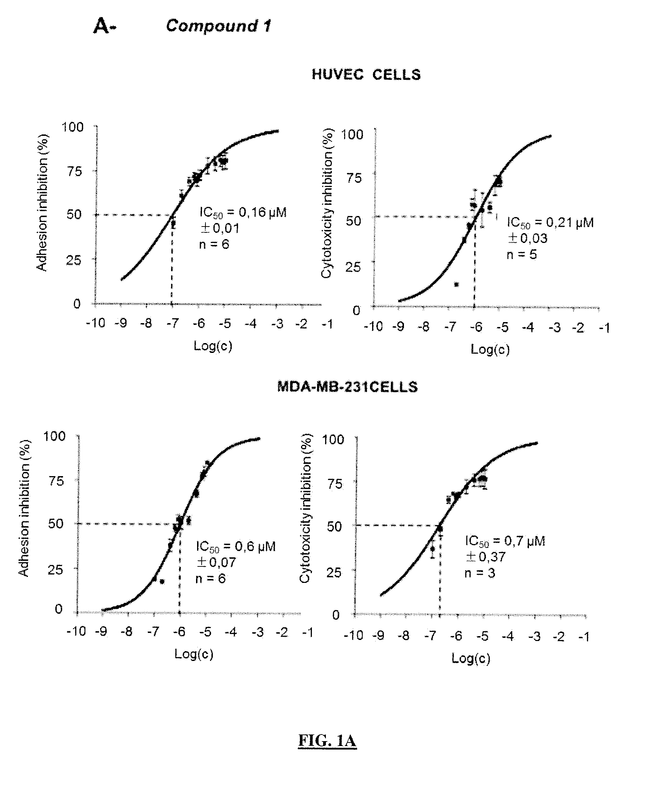

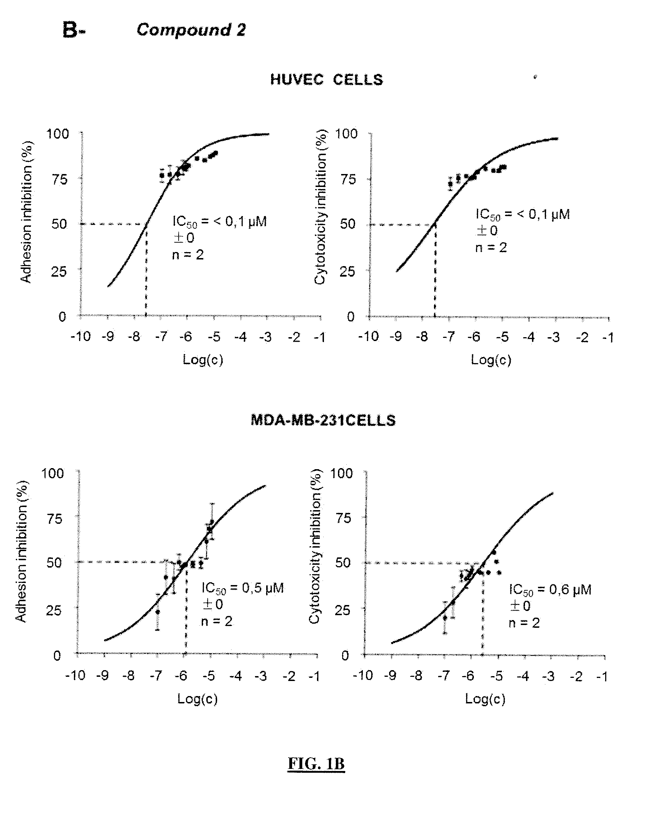

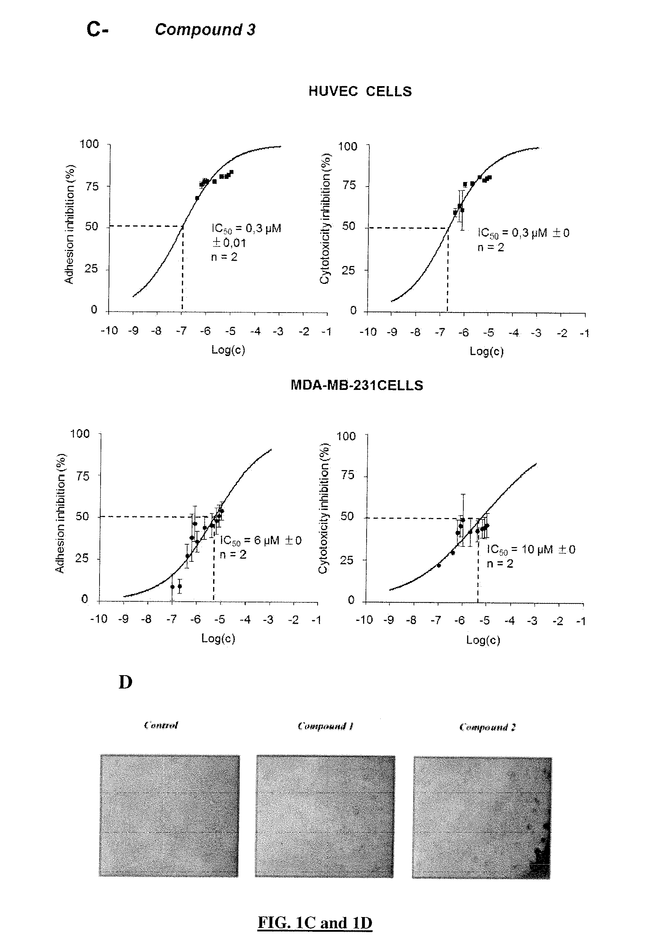

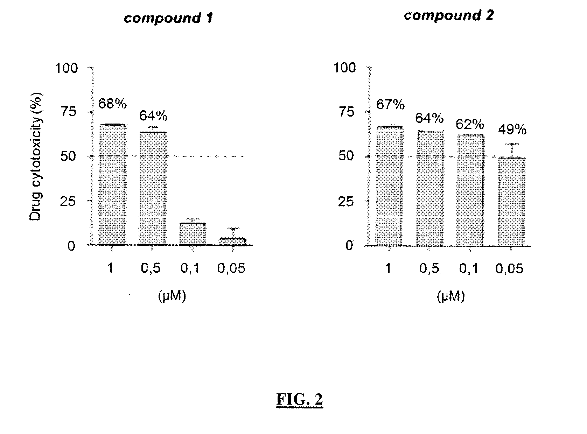

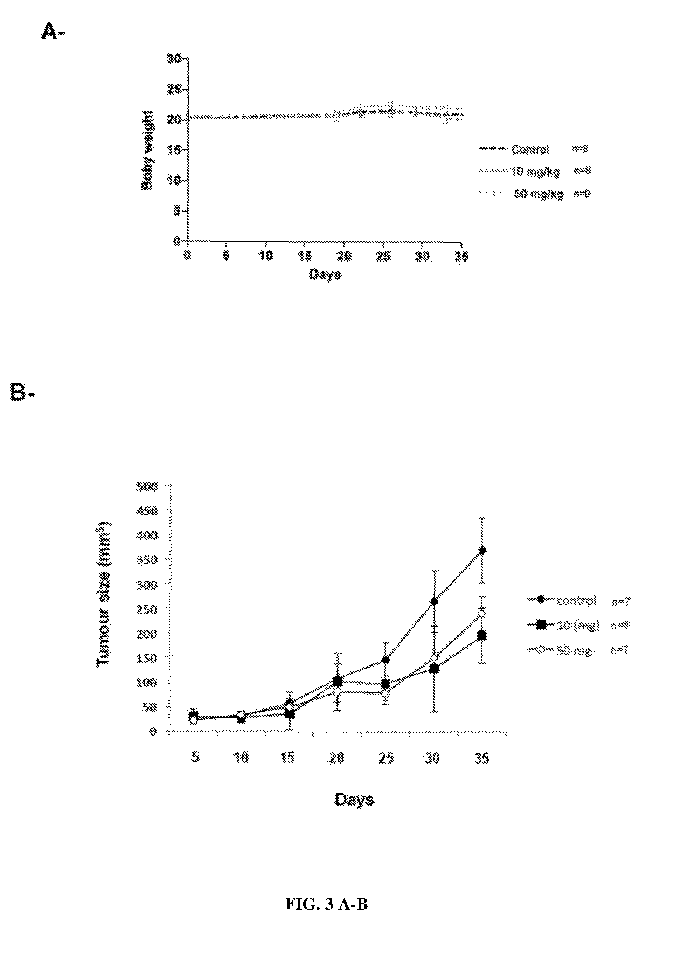

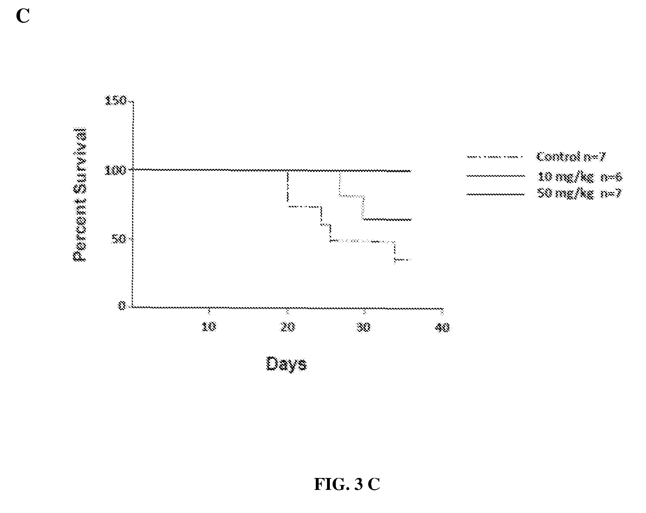

The Inventors surprisingly showed that the compounds of the invention were cytotoxic for cancer cell lines of different origins (Figures 1 and 2; Table 1), and that they inhibited the adhesion of cancer cells (Figures 1A to 1C). Moreover, the Inventors demonstrated that the compounds of the invention were efficient in vivo as anti-cancer agents (Figure 3). Indeed, the administration to mice of the compounds of the invention inhibited the expansion of a tumor, and resulted in a prolonged survival of the mice.

In one embodiment, the pharmaceutical composition of the invention may be used for inhibiting the growth of a tumor. In one embodiment, the pharmaceutical composition of the invention may be used for inhibiting the number and the development of cancerous cells within the body of the subject. In one embodiment, the pharmaceutical composition of the invention may be used for inhibiting the occurrence of metastases.

According to an embodiment, cancer is solid tumor, metastatic cancer or non-metastatic cancer.

In one embodiment, the cancer may originated in the bladder, blood, bone, bone marrow, brain, breast, cervic area, colon, esophagus, eye and periocular including subconjunctival tissues, duodenum, small intestine, large intestine, rectum, anus, gum, head, kidney, liver, lung, nasopharynx, neck, ovary, pancreas, prostate, skin, stomach, testis, tongue, or uterus.

Examples of cancer include, but are not limited to, carcinoma, adenocarcinoma, lymphoma, blastoma, hepatoma, sarcoma, and leukemia. More particular examples of such cancers include squamous cell cancer, lung cancer (including small-cell lung cancer, non-small cell lung cancer, adenocarcinoma of the lung, and squamous carcinoma of

the lung), cancer of the peritoneum, hepatocellular cancer, gastric or stomach cancer (including gastrointestinal cancer), pancreatic cancer, such as, for example, pancreatic carcinoma, glioblastoma, cervical cancer, ovarian cancer, liver cancer, bladder cancer, hepatoma, breast cancer, colon cancer, such as, for example, colon adenocarcinoma (including a colon adenocarcinoma grade II), colorectal cancer, such as, for example, colorectal carcinoma, endometrial or uterine carcinoma, salivary gland carcinoma, kidney or renal cancer, liver cancer, prostate cancer, such as, for example, prostate adenocarcinoma, vulval cancer, thyroid cancer, osteosarcoma, neuroblastoma, hepatic carcinoma and various types of head and neck cancer, as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

According to an embodiment, the cancer is breast cancer, cervical cancer, pancreatic carcinoma, colorectal carcinoma, colon adenocarcinoma, such as, for example, colon adenocarcinoma grade II, osteosarcoma, prostate cancer, such as, for example, prostate adenocarcinoma, gioblastoma or neuroblastoma.

According to an embodiment, the pharmaceutical composition of the invention is injected, preferably systemically injected. Examples of formulations adapted to systemic injections include, but are not limited to, liquid solutions or suspensions, solid forms suitable for solution in, or suspension in, liquid prior to injection. Examples of systemic injections include, but are not limited to, intravenous, subcutaneous, intramuscular, intradermal and intraperitoneal injection, and perfusion.

According to another embodiment, the composition of the invention is intratumorally or intralesionally injected.

According to an embodiment, when injected, the pharmaceutical composition of the invention is sterile. Methods for obtaining a sterile pharmaceutical composition include, but are not limited to, GMP synthesis (GMP stands for "Good manufacturing practice").

According to another embodiment, the pharmaceutical composition of the invention is orally administered. Examples of formulations adapted to oral administration include, but are not limited to, solid forms, liquid forms and gels. Examples of solid forms adapted to oral administration include, but are not limited to, pill, tablet, capsule, soft gelatin capsule, hard gelatin capsule, caplet, compressed tablet, cachet, wafer, sugar-coated pill, sugar coated tablet, orodispersing/orodisintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

According to another embodiment, the pharmaceutical composition of the invention is administered topically. Topical administration may be particularly advantageous for the treatment of skin cancers. Examples of formulations adapted to topical administration include, but are not limited to, patch, such as, for example, transdermic patch, ointment, gel, cream and the like.

According to another embodiment, for the treatment of conditions of the lungs or of the respiratory tract, aerosol delivery can be used.

Other examples of administration routes include, but are not limited to, nasal, buccal, rectal, vaginal, intratracheal, endoscopic and percutaneous administration.

According to an embodiment, the pharmaceutical composition of the invention is administered preoperatively, and/or postoperatively.

According to an embodiment, the pharmaceutical composition of the invention is packaged in unitary dosages. Examples of unitary dosages include, but are not limited to, vials, syringe, pill, caplet, capsule, tablet, sealed phial and pouch containing the composition of the invention.

Examples of pharmaceutically acceptable vehicles which could be used in the pharmaceutical composition of the invention are well known from the one skilled in the art. The selection of a specific vehicle is dependent on the administration route and on the formulation of the composition of the invention.

Examples of pharmaceutically acceptable vehicles include, but are not limited to water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol™, vegetable oils and the like.

In one embodiment, the subject receiving the pharmaceutical composition of the invention is an animal, including a human.

According to a first embodiment, the subject receiving the pharmaceutical composition of the invention is diagnosed with a cancer.

According to a second embodiment, the subject receiving the pharmaceutical composition of the invention underwent a surgical operation to remove a tumor.

According to a third embodiment, the subject receiving the pharmaceutical composition of the invention was

previously treated with an anti-cancer treatment, or is simultaneously treated with an anti-cancer treatment, or is planned to be treated with an anti-cancer treatment. Examples of anti-cancer treatment include, but are not limited to, chemotherapy and radiotherapy.

In the sense of the present invention, these three embodiments are not exclusive: a subject may be concerned by one, two or three of these embodiments.

According to an embodiment, the pharmaceutical composition of the invention comprises from about 0.1 mg to about 50 g of the compound of general formula (I) of the invention.

According to an embodiment, the pharmaceutical composition is such that an amount of the compound of general formula (I) of the invention ranging from 1 μg/kg of body weight to 1 g/kg of body weight is administered to the subject.

The present invention also relates to a method for preventing and/or treating a cancer in a subject in need thereof, wherein the method comprises the administration of a pharmaceutical composition comprising a therapeutically effective amount of a compound of general formula (I) to the subject.

According to an embodiment, the method of the invention comprises the injection of the pharmaceutical composition of the invention, preferably the systemic injection. Examples of systemic injections include, but are not limited to, intravenous, subcutaneous, intramuscular, intradermal and intraperitoneal injection, and perfusion. According to another embodiment, the composition of the invention is intratumorally or intralesionally injected.

According to another embodiment, the method of the invention comprises the oral administration of the pharmaceutical composition of the invention.

According to another embodiment, the method of the invention comprises the topical administration of the pharmaceutical composition of the invention. According to another embodiment, the method of the invention comprises the administration of the pharmaceutical composition of the invention by the use of an aerosol.

Other examples of administration routes which could be used in the method of the invention include, but are not limited to, nasal, buccal, rectal, vaginal, intratracheal, endoscopic and percutaneous administration.

According to an embodiment, the method of the invention comprises the preoperative or postoperative administration of the pharmaceutical composition of the invention.

In one embodiment, the subject concerned by the method of the invention is an animal, including a human.

According to a first embodiment, the subject concerned by the method of the invention is diagnosed with a cancer. According to a second embodiment, the subject concerned by the method of the invention underwent a surgical operation to remove a tumor. According to a third embodiment, the subject concerned by the method of the invention was previously treated with an anti-cancer treatment, or is simultaneously treated with an anti-cancer treatment, or is planned to be treated with an anti-cancer treatment. Examples of anti-cancer treatment include, but are not limited to, chemotherapy and radiotherapy. In the sense of the present invention, these three embodiments are not exclusive: a subject may be concerned by one, two or three of these embodiments.

The present invention thus also relates to a pharmaceutical composition comprising a therapeutically effective amount of compound of general formula (I) in association with a pharmaceutically acceptable vehicle for preventing and/or treating a disease, disorder or condition related to angiogenesis in a subject in need thereof. In the present invention, a disease, disorder or condition related to angiogenesis may also be referred as an angiogenic disease, disorder or condition.

The Inventors herein present evidences that the compounds of the invention inhibited the formation of neovessels (Figure 1D). Surprisingly, they also showed that the compounds of the invention inhibited the adhesion of HUVEC cells (endothelial cells) and that they are cytotoxic for these cells (Figures 1A, 1B and 1C), thus demonstrating an anti-angiogenic effect.

According to an embodiment, the pharmaceutical composition of the invention is for use for inhibiting the formation of neovessels. According to another embodiment, the pharmaceutical composition of the invention is for use for inhibiting the adhesion of endothelial cells. According to another embodiment, the pharmaceutical composition of the invention is for use for inhibiting the growth, development or division of endothelial cells.

According to an embodiment, the disease, disorder or condition related to angiogenesis is tumor related. Indeed, the growth and metastasis of tumors are directly linked to angiogenesis: the tumor may stimulate the growth of neovessels, which (i) allow the supply of nutrients and oxygen necessary to its growth, and (2) are escape routes for tumors, facilitating the dissemination of metastatic cells through the blood circulation. Therefore, in one embodiment, the disease, disorder or condition related to angiogenesis is a cancerous or non-cancerous tumor or a metastasis of a tumor. Examples of cancers and of cancerous tumors are listed here-above. Examples of non-cancerous tumors include, but are not limited to, angiofibroma and hemangiomas.

According to an embodiment, the disease, disorder or condition related to angiogenesis is an ocular disease, disorder or condition.

In one embodiment, the ocular disease, disorder or condition related to angiogenesis is associated with retinal, peripheral retinal and/or choroidal neovascularization. Examples of such angiogenic diseases include, but are not limited

to uveitis, choroiditis, choroidal vasculopathy (including polypoidal choroidal vasculopathy), hypersensitive retinopathy, retinochoroiditis, chorioretinitis, retinal angiomatosis, retinal degeneration, macular degeneration, age related macular degeneration (AMD, including wet and dry AMD), retinal detachment, retinal neovascularisation, proliferative vitreoretinopathy, retinopathy of prematurity (ROP), central serous chorioretinopathy, diabetic retinopathy (including nonproliferative and proliferative diabetic retinopathy), posterior segment trauma, retinal vascular pathologies, retinal telangiectesa, endophthalmitis, macular edema, radiation-induced retinopathy, cystoid macular edema, macular dystrophy (including vitelliform macular dystrophy), diabetic retinopathy, inflammatory pathologies of the retina, ischemic vascular disease, sickle cell anemia, sickle cell retinopathy, sarcoid, syphilis, pseudoxanthoma elasticum, Pagets disease, vein occlusion, artery occlusion, carotid obstructive disease, chronic uveitis/vitritis, fundus flavimaculatus, mycobacterial infections, Lyme's disease, systemic lupus erythematosis, systemic pathologies with implications for the retina, Eales disease, Bechets disease, infections causing a retinitis or choroiditis, presumed ocular histoplasmosis, Bests disease, myopia, optic pits, Stargarts disease, pars planitis, chronic retinal detachment, hyperviscosity syndromes, toxoplasmosis, toxocariasis, rubella, Vogt-Koyanagi-Harada syndrome, multifocal choroiditis, retinitis pigmentosa, retinischisis, trauma, proliferative diabetic retinopathy, branch retinal vein occlusion, branch retinal arteriolar occlusion, carotid cavernous fistula, sickling hemoglobinopathy (including SS hemoglobinopathy and SC hemoglobinopathy), non-sickling hemoglobinopathy (including AC hemoglobinopathy and AS hemoglobinopathy), IRVAN syndrome (retinal vasculitic disorder characterized by idiopathic retinal vasculitis, an aneurysm, and neuroretinitis), retinal embolization, familial exudative vitreoretinopathy, hyperviscosity syndrome (including leukemia, Waldenstrom macroglobulinemia, multiple myeloma, polycythemia or myeloproliferative disorder), aortic arch syndrome, Eales disease and post-laser complications.

In one embodiment, the ocular disease, disorder or condition related to angiogenesis is associated with corneal neovascularization. Examples of such angiogenic diseases include, but are not limited to diabetic retinopathy, retinopathy of prematurity, corneal graft rejection, neovascular glaucoma and retrolental fibroplasia, epidemic keratoconjunctivitis, Vitamin A deficiency, contact lens overwear, atopic keratitis, superior limbic keratitis, pterygium keratitis sicca, sjogrens, acne rosacea, phylectenulosis, syphilis, Mycobacteria infections, lipid degeneration, chemical bums, bacterial ulcers, fungal ulcers, Herpes simplex infections, Herpes zoster infections, protozoan infections, Kaposi sarcoma, Mooren ulcer, Terrien's marginal degeneration, marginal keratolysis, trauma, rheumatoid arthritis, systemic lupus, polyarteritis, Wegeners sarcoidosis, Scleritis, Steven's Johnson disease, periphigoid radial keratotomy, and corneal graph rejection.

In one embodiment, the ocular disease, disorder or condition related to angiogenesis is selected from the group comprising diseases associated with rubeosis (neovascularization of the angle) and diseases caused by the abnormal proliferation of fibrovascular or fibrous tissue including all forms of proliferative vitreoretinopathy, whether or not associated with diabetes.

According to an embodiment, the disease, disorder or condition related to angiogenesis is a disease of the renal and/or urogenital tract. Examples of angiogenic diseases of the renal and/or urogenital tract include, but are not limited to, diabetic nephropathy, endometriosis, haemolytic uremic syndrome, hypertensive nephrosclerosis, inflammatory renal disease, such as glomerulonephritis (including mesangioproliferative glomerulonephritis), inhibition of ovulation and corpus luteum formation, inhibition of implantation and inhibition of embryo development in the uterus and nephrotic syndrome.

According to an embodiment, the disease, disorder or condition related to angiogenesis is a disease of the digestive tract. Examples of angiogenic diseases of the digestive tract include, but are not limited to, inflammatory bowel disease.

According to an embodiment, the disease, disorder or condition related to angiogenesis is a disease of the respiratory tract. Examples of angiogenic diseases of the respiratory tract include, but are not limited to, sarcoid, sarcoidosis, Wegeners sarcoidosis, lung inflammation and chronic asthma.

According to an embodiment, the disease, disorder or condition related to angiogenesis is a skin disease. Examples of angiogenic skin diseases include, but are not limited to, psoriasis, acne rosacea, Kaposi's sarcoma, leukemia, Osier-Weber syndrome (Osler-Weber-Rendu), pemphigoid, pseudoxanthoma elasticum, pyogenic granuloma, scleroderma, Steven's Johnson disease and syphilis.

According to an embodiment, the disease, disorder or condition related to angiogenesis is an autoimmune disease. Examples of autoimmune diseases related to angiogenesis include, but are not limited to, chronic asthma, Crohn's disease, pemphigoid, scleroderma, Sogrens syndrome and systemic lupus.

According to an embodiment, the disease, disorder or condition related to angiogenesis is consecutive to a graft. Examples of reactions related to angiogenesis following the graft of an organ or tissue include, but are not limited to, immune rejection of transplanted organs or tissues (including corneal graft rejection), corneal graft neovascularization and diseases occurring after transplants.

According to an embodiment, the disease, disorder or condition related to angiogenesis is a post-surgery reaction, a hyperproliferation or a hypertrophy. Examples of such diseases include, but are not limited to, abnormal proliferation of fibrovascular tissue, hypertrophic scars and hypertrophy following surgery.

According to an embodiment, the disease, disorder or condition related to angiogenesis is a disease of bones

or joints. Examples of diseases of bones or joints related to angiogenesis include, but are not limited to, Pagets disease, osteoarthritis, hemophilic joints, psoriatic arthritis, rheumatoid arthritis and nonunion fractures.

According to an embodiment, the disease, disorder or condition related to angiogenesis is abnormal wound healing.

According to an embodiment, the disease, disorder or condition related to angiogenesis is chronic inflammation.

According to an embodiment, the disease, disorder or condition related to angiogenesis is a disease of the cardiovascular system or of blood. Examples of diseases of the cardiovascular system or of blood related to angiogenesis include, but are not limited to, arterial arteriosclerosis, arteriovenous malformations, artery occlusion, atherosclerosis, carotid obstructive disease, diseases and disorders characterized by undesirable vascular permeability, e.g., edema associated with brain tumors, ascites associated with malignancies, hyperviscosity syndromes, pericardial effusion, such as that associated with pericarditis, and pleural effusion, polyarteritis, preeclampsia, vascular adhesions and vein occlusion.

According to an embodiment, the disease, disorder or condition related to angiogenesis is an infection. Examples of infections causing angiogenic disorders include, but are not limited to, bacterial infection, bacterial ulcers, fungal infection, fungal ulcers, Herpes simplex infections, Herpes zoster infections, Lyme's disease, Mycobacteria infections other than leprosy, parasitic diseases, protozoan infections, toxoplasmosis and viral infection.

According to an embodiment, the pharmaceutical composition of the invention may be administered by injection, oral administration, topical administration, implant, aerosol, or by nasal, buccal, vaginal, rectal, intratracheal, endoscopic or percutaneous administration.

According to an embodiment, the pharmaceutical composition of the invention is injected. Examples of formulations adapted to injections include, but are not limited to, liquid solutions or suspensions, solid forms suitable for solution in, or suspension in, liquid prior to injection. Examples of injections include, but are not limited to, systemic, intravenous, intraocular, subcutaneous, intramuscular, intradermal, intraperitoneal, intratumoral and intralesional injection, and perfusion. According to an embodiment, when injected, the pharmaceutical composition of the invention is sterile. Methods for obtaining a sterile pharmaceutical composition include, but are not limited to, GMP synthesis (GMP is for "Good manufacturing practice").

According to another embodiment, the pharmaceutical composition of the invention is orally administered. Examples of formulations adapted to oral administration include, but are not limited to, solid forms, liquid forms and gels. Examples of solid forms adapted to oral administration include, but are not limited to, pill, tablet, capsule, soft gelatin capsule, hard gelatin capsule, caplet, compressed tablet, cachet, wafer, sugar-coated pill, sugar coated tablet, orodispersing/orodisintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

In one embodiment of the invention, the disease, disorder or condition is an ocular disease. According to this embodiment, the pharmaceutical composition of the invention may be injected, for example intraocularly or periocularly injected. By "intraocularly injected" is meant, in the present invention, injected directly in the interior of the eye, wherein the interior of the eye means any area located within the eyeball, and which generally includes, but is not limited to, any functional (e.g. for vision) or structural tissues found within the eyeball, or tissues or cellular layers that partially or completely line the interior of the eyeball. Specific examples of such areas include the anterior chamber, the posterior chamber, the vitreous cavity, the choroid, the macula, and the retina, and blood vessels and nerves which vascularize or innervate a posterior ocular region or site. By "periocularly injected" is meant, in the present invention, injected in the tissues adjacent to the eyeball, such as, for example, peribulbar, laterobulbar, subconjonctival, sub-tenon or retrobulbar injection. Examples of formulations adapted to intraocular or periocular injection include, but are not limited to, solutions, emulsions or suspensions. According to this embodiment, the pharmaceutical composition of the invention may also be topically applied. Examples of formulations adapted to topical administration to the eye include, but are not limited to, ointments and eye drops. According to this embodiment, the pharmaceutical composition of the invention may also be an intraocular implant. Examples of intraocular implant include, but are not limited to, intraocular lenses.

According to another embodiment, the pharmaceutical composition of the invention is administered topically. Topical administration may be particularly advantageous for the treatment of skin conditions. Examples of formulations adapted to topical administration include, but are not limited to, patch, such as, for example, transdermic patch, ointments, eye drops, gels, cream and the like.

According to another embodiment, the pharmaceutical composition of the invention is administered by an aerosol. Treatments using an aerosol are of particular interest for the treatment of conditions of the lungs or of the respiratory tract.

According to an embodiment, the pharmaceutical composition of the invention is a suppository. The use of suppositories is of particular interest for the treatment of diseases of the urogenital tract and/or of the digestive tract.

According to an embodiment, the pharmaceutical composition of the invention is packaged in unitary dosages. Examples of unitary dosages include, but are not limited to, vials, syringe, pill, caplet, capsule, tablet, sealed phial,

suppositories, and pouch containing the composition of the invention.

According to an embodiment, the pharmaceutical composition of the invention is administered preoperatively, and/or postoperatively.

Examples of pharmaceutically acceptable vehicles which could be used in the pharmaceutical composition of the invention are well known from the one skilled in the art. The selection of a specific vehicle is dependent on the administration route and on the formulation of the composition of the invention.

Examples of pharmaceutically acceptable vehicles include, but are not limited to water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol™, vegetable oils and the like.

In one embodiment, the subject receiving the pharmaceutical composition of the invention is an animal, including a human.

According to an embodiment, the subject receiving the pharmaceutical composition of the invention is diagnosed with a disease, disorder or condition related to angiogenesis.

According to a second embodiment, the subject receiving the pharmaceutical composition of the invention was previously treated with another anti-angiogenic treatment, or is simultaneously treated with another anti-angiogenic treatment, or is planned to be treated with another anti-angiogenic treatment.

In the sense of the present invention, these two embodiments are not exclusive: a subject may be concerned by only one or two of these embodiments.

According to an embodiment, the pharmaceutical composition of the invention comprises from about 0.1 mg to about 50 g of the compound of formula (I) of the invention.

According to an embodiment, the pharmaceutical composition is such that an amount of the compound of formula (I) of the invention ranging from 1 μg/kg of body weight to 1 g/kg of body weight is administered to the subject.

The present invention also relates to a method for preventing and/or treating an angiogenic disease, disorder or condition in a subject in need thereof, wherein the method comprises the administration of a pharmaceutical composition comprising a therapeutically effective amount of a compound of general formula (I) to the subject.

According to an embodiment, the method of the invention comprises the injection, the oral, topical, nasal, buccal, vaginal, rectal, intratracheal, endoscopic or percutaneous administration, or the administration by the use of an implant or of an aerosol of the pharmaceutical composition of the invention.

In one embodiment, the subject concerned by the method of the invention is an animal, including a human.

According to an embodiment, the subject concerned by the method of the invention is diagnosed with a disease, disorder or condition related to angiogenesis. According to a second embodiment, the subject concerned by the method of the invention was previously treated with another anti-angiogenic treatment, or is simultaneously treated with another anti-angiogenic treatment, or is planned to be treated with another anti-angiogenic treatment. In the sense of the present invention, these two embodiments are not exclusive: a subject may be concerned by only one or two of these embodiments.

According to an embodiment, the method of the invention comprises the preoperative and/or the postoperative administration of the pharmaceutical composition of the invention.

## BRIEF DESCRIPTION OF THE DRAWINGS

**Figure 1** is a combination of graphs and pictures showing the in vitro effect of the compounds **1, 2** and **3** of the invention on cell adhesion, cell survival and vessel formation. (A) Graphs showing the effect of the compound **1** of the invention on the adhesion and on the survival of HUVEC and MDAMB-231 cells. (B) Graphs showing the effect of the compound **2** of the invention on the adhesion and on the survival of HUVEC and MDAMB-231 cells. (B) Graphs showing the effect of the compound **3** of the invention on the adhesion and on the survival of HUVEC and MDAMB-231 cells. (D) Images showing the effect of the compounds **1** and **2** of the invention on the formation of vessels.

**Figure 2** is graphs showing the inhibitory effect of the compounds **1** and **2** of the invention on HeLa cells following cytotoxicity.

**Figure 3** is a combination of graphs showing the in vivo efficiency of the compound **1** of the invention. (A) Effect of the compound **1** on body weight. (B) Effect of the compound **1** on tumor growth. (C) Kaplan-Meier survival curves showing the effect of the compound **1** on survival of mouse.

## EXAMPLES

### Example 1: Synthesis of the compounds of the invention

Synthesis of N-[5-(1H-benzimidazol-2-yl)-2-methylphenyl]-N'-(2,3-dihydro-1,4-benzodioxin -6-ylcarbonyl)thiourea (compound **1** of the invention)

A solution of 4-Methyl-3-nitrobenzaldehyde (3.34g, 20.2 mmol, leq) and sodium bisulfate (11.51g, 60.6 mmol, 3eq) in 10 ml of dimethylformamide (DMF), was refluxed for 1hr, then a solution of o-phenediamine (2.18g, 20.2 mmol, leq) in 10 ml of DMF was added drop wise and the mixture was refluxed for a further 3hour. The solution was cooled to room temperature and poured into ice-water. The organic layer was then washed with brine and dried over $Na_2SO_4$ then evaporated to dryness. The residue obtained was triturated in order to eliminate impurities and the solid was collected by filtration to give 5(1H-benzoimidazol-2-yl)-2-methyl-phenylamine as a brown powder (3.04g, yield 67%). NMR (DMSO-d6): 2.14 (s, 3H, $CH_3$); 5.07 (s, 2H, $NH_2$); 7.1-7.6 (m, 7H, H-Ar); 12.65 (s, 1H, NH).

1,4-Benzodioxane-6-carboxylic acid (4.5g, 25 mmol) was refluxed in thionyl chloride (25ml) for 3hr, the solution was then evaporated to dryness to give 1,4-benzodioxin-6-carbonyl chloride as a pale solid 4.9g (yield: 100%).

A solution of benzodioxin-6-carbonyl chloride (2.2g, 10 mmol, leq) and ammonium thiocyanate (0.84g, 10 mmol, leq) in dry 8 ml acetone was refluxed for 1hr. The mixture was then cooled to room temperature and a solution of 5(1H-benzoimidazol-2-yl)-2-methyl-phenylamine (2.34g, 10 mmol, leq) in 18 ml dry acetone was added. The mixture was refluxed for another 1hr, then the solution was cooled and 100ml of water was added. The precipitate was collected by filtration and the crude product was recrystalized in EtOH to obtained 1 white powder (0.870g, yield 63%). NMR (DMSO-d6): 2.34 (s, 3H, $CH_3$); 4.34 (s, 4H, 2*$CH_2O$); 7.0-8.1 (m, 10H, H-Ar); 8.34 (s, 1H, NH); 11.54 (s, 1H, NH); 12.40 (s, 1H, NH). MS (ESI+): 445 (m+$H^+$) found for 444 calculated. HPLC (Vydac C18, 4.6*250mm. A: H2O with 0.1% TFA, B: 70% acetonitrile with 0.09% TFA. Flow 1ml/min): Rt: 14.9 min.

Synthesis of N-[3-(1H-benzimidazol-2-yl)phenyl]-N'-(2,3-dihydro-1,4-benzodioxin -6- ylcarbonyl)thiourea (compound **2** of the invention):

A solution of 3-nitrobenzaldehyde (2.90g, 26.5 mmol, leq) and sodium bisulfate (15.10g, 79.5 mmol, 3eq) in 10 ml of DMF, was refluxed for 1hr, then a solution of o-phenediamine (4.00g, 26.5 mmol, leq) in 10 ml of DMF was added drop wise and the mixture was refluxed for a further 3hour. The solution was cooled to room temperature and poured into ice-water. The organic layer was then washed with brine and dried over $Na_2SO_4$ then evaporated to dryness. The residue obtained was triturated in order to eliminate impurities and the solid was collected by filtration to give 5(1H-benzoimidazol-2-yl)-2-methylphenylamine as a brown powder (2.00g, yield 39%). NMR (DMSO-d6): 6.07 (s, 2H, $NH_2$); 7.2-7.6 (m, 8H, H-Ar); 12.6 (s, 1H, NH).

A solution of benzodioxin-6-carbonyl chloride (0.97g, 4.4 mmol, leq) and ammonium thiocyanate (0.37g, 4.4 mmol, leq) in dry 6 ml acetone was refluxed for 1hr. The mixture was then cooled to room temperature and a solution of 5(1H-benzoimidazol-2-yl)-2-methyl-phenylamine (0.96g, 4.4 mmol, leq) in 15 ml dry acetone was added. The mixture was refluxed for another 1hr, then the solution was cooled and 100ml of water was added. The precipitate was collected by filtration and the crude product was recrystalized in EtOH to obtained 2 white powders (0.465g, yield 27%). NMR (DMSO-d6): 4.33-4.37 (m, 4H, 2*$CH_2O$); 7.00-8.12 (m, 11H, H-Ar); 8.41 (s, 1H, NH); 11.58 (s, 1H, NH); 12.86 (s, 1H, NH). MS (ESI+): 431 ($m+H^+$) found for 430 calculated. HPLC (Vydac C18, 4.6*250mm. A: H2O with 0.1% TFA, B: 70% acetonitrile with 0.09% TFA. Flow 1ml/min): Rt: 13.8 min.

**Example 2: Characterisation of the compounds 1, 2 and 3 of the invention**

| Product | LogP value | MS | Melting point |
|---|---|---|---|
| (1) | 3.15 | MH+445 | 202˚C |
| (2) | 2.94 | MH+431 | 212˚C |
| (3) | 3.061 | MH+417 | 228˚C |

LogP values were calculated using jlogP program, available at the web address: http://www.vls3d.com/programs.html#section9.

**Example 3: Biological examples**

**Methods**

***Cell culture***

HUVEC (Lonza, Switzerland, cat n˚: C2517A) were maintained in EBM®-2 (*Lonza*, *France,* cat n˚: CC-3162) supplemented with a kit including 2% foetal bovine serum, human fibroblast growth factor-2 (hFGFB), human epidermal growth factor (hEGF), insulin like growth factor-1 (R3-IGF-1), hydrocortisone, gentamicin and amphotericin-B, heparin and ascorbic acid (Lonza, France, cat n˚: CC-4176).

Several cancer cell lines were used: breast cancer cells (MDA-MB-231 (ATTCC® Number HTB-26™), MDA-MB-436 (ATTCC® Number HTB-130),™ MDA-MB-438, BT-474 (ATTCC® Number HTB-20™)); cervical cancer cells (HeLa (ATTCC® Number CCL-2™)); colon cancer cells (HCT-116 (ATTCC® Number CCL-247 ™) and HT-29 (ATTCC® Number HTB-38™)); osteosarcoma cells (U20S (ATTCC® Number HTB-96™)); pancreas cancer cells (MIA PaCa-2 (ATTCC® Number CRL- 1420™) and Panc-10.5 (ATTCC® Number CRL-2547T™)); prostate cancer cells (PC-3 (ATTCC® Number CRL-1435™) and LNCaP (ATTCC® Number CRL-1740™)); brain cancer cells (U87 glioblastoma (ATTCC® Number HTB-14™) and SH-SYSY neuroblastoma (ATTCC® Number CRL-2266™)).

All the cancer cell lines were maintained in cultured in DMEM GlutaMAX™ (GIBCO Invitrogen, France, cat n°: 31966047) enhanced with 10% foetal bovine serum (GIBCO Invitrogen, France, cat n°: 10270-106) and 1% penicillin G/streptomycin (GIBCO Invitrogen, France, cat n°15140-122). Only Panc-10.5 cells were cultivated in RPMI 1640 medium (Gibco) supplemented with 2 mM L-glutamine, 1.5 g/L sodium bicarbonate, 4.5 g/L glucose, 10 mM HEPES, 1 mM sodium pyruvate, 10 Units/ml human insulin, 85% fetal bovine serum (SVF) : 15% (75 ml in 500 ml of medium).

Cells were grown as subconfluent monolayer and cultured in 75cm$^2$ flasks at 37°C, 5% $CO_2$ in a humidified atmosphere, and medium was changed every two days. Only cells from passages 2-6 were used for the experiments.

After observation of cells under the microscope, old media were removed and the adherent cells washed gently twice with PBS (Phosphate Buffer Saline, -$Ca^{2+}$, -$Mg^{2+}$, Lonza cat n°: 17-512F/12). All cells were detached with 3 ml of trypsin/EDTA (chelator of calcium and magnesium ions) (Lonza), incubated for 5 min a 37°C, and examined using a microscope to ensure that the cells were detached and floating. Then, 3 ml TNS (Trypsin Neutralizing Solution, Lonza, France, cat n°CC-5002) or of culture medium were added to inactivate the trypsin, and the cell suspension was transferred in a falcon and centrifuged for 7 min at 1400 rpm. The supernatant was removed and the cell pellet resuspended in 10 μl of culture medium. 50 μl of this solution were transferred in 20 μl of isotonic solution and the cells were counted by count cellular (Beckman Coulter). At day 0, 200 μl of cellular suspension (3.10$^3$ cells/well for the HUVEC cells, breast cancer cells (MDA-MB-231 1.10$^4$ cells/well, MDA-MB-436 1.10$^4$ cells/well, MDA-MB-438 2.10$^4$ cells/well, BT-474 2.5.10$^4$ cells/well); cervical cancer cells (HeLa 5.000 cells/well); colon cancer cells (HCT-116 5.10$^3$ cells/well, HCT-29 1.1.10$^4$ cells/well); osteosarcoma cells (U2OS 8.10$^3$ cells/well); pancreas cancer cells (MIA PaCa-2 5.10$^3$ cells/well, Panc-10.5 1.5.10$^4$ cells/well); prostate cancer cells (PC-3 4.10$^3$ cells/well, LNCaP 2.10$^4$ cells/well); brain cancer cells (U87 5.10$^3$ cells/well, SH-SYSY 8.10$^4$ cells/well)) were then plated on 96 well plates and incubated at 37°C in a humidified atmosphere of 5% $CO_2$ for 24 hours.

At day 1, the compounds **1** or **2** of the invention were added at increasing concentrations (0.1 - 10 μM) in the wells and plates were incubated at 37 °C in a humidified atmosphere of 5% $CO_2$ for 72 hours.

***Adhesion test***

The crystal violet (hexamethyl pararosaniline chloride) is used as biologic colorant. It enters in the living cells, allowing the visualisation of the dark purple adherent living cells on the bottom of the plate.

After an incubation period of 72 hours, the adherent cells were fixed with 50ml paraformaldehyde 3% for 15 min at room temperature. The medium and non-adherent cells were removed, and the cells were washed extensively with 100 ml PBS (+$Ca^{2+}$ and +$Mg^{2+}$) (Lonza) and then stained with 50 μl of crystal violet 0.04% for 30 min at room temperature. The cells were washed gently 3 times with 100 μl PBS and were permeabilised with 100 μl triton 2% for 15 min. The microplate was stirred to obtain a homogenous solution and absorbance was quantified at 580 nm by Microplate Manager 5.2.

The percentage of inhibition is calculated as follow: % inhibition = 100- [($DO_{sample}$/ $DO_{control}$) * 100].

***Proliferation assay***

The cell proliferation WST-1 assay is a colorimetric assay for the quantification of cell proliferation and viability, based on the enzymatic cleavage of the tetrazolium salt WST-1 added to the culture medium, to a water soluble colored formazan dye, detected by absorbance at 450 nm, by mitochondrial succinate-tetrazolium reductase in viable cells. An augmentation in enzyme activity will then lead to an increase in the amount of formazan dye formed, which directly correlates to the number of metabolically active cells in the culture.

After an incubation period of 72 hours, at day 4, were prepared the 19 ml medium with reagent WST-1 1ml (10 ml WST-1 in 200 ml well). The medium was removed from the 96 microplate and 200 μl of solution WST-1/medium were added for each well. The cells were incubated at 37 °C in a humidified atmosphere of 5% $CO_2$ for 1 hour and 30 min and the absorbance at 450-655nm was measured by Microplate Manager 5.2.

***Inhibition of VEGF on NRP-1 or VEGF-R1 interaction***

Elisa test was performed covering a 96-well microplate (BD Biosciences, France, cat n°:353046) surface with

100 μl of 1X PBS, pH 7.4 with $Ca^{2+}$ and $Mg^{2+}$, (Lonza, France, cat n°: 17-513F/12) solution containing 0.2 μg/ml of VEGF-R1 (R&D Systems, UK, cat n°: 321-FL-050/CF) or 2 μg/ml of NRP-1 (R&D Systems, UK, cat n°: 3870-N1-025). The plate was sealed and incubated overnight at 4°C. After 3 washes with 200 μl of 1X PBS 0.5% (v/v) Tween 20 (Sigma, France, cat n°34078) (Buffer A), the plate was blocked by adding 200 μl of 1× PBS with 0.5% (w/v) BSA (Eurobio, France, cat n°GAUBSA01-65) and agitated at 37°C for 2 hours. After washing five times with Buffer A, 50 μl/well of the compounds of the invention at 10μM were added for the specific signal and 50 μl/well for the non-specific and max binding signal and incubated at 37°C for 1 hour, shacking. 50 μl of btVEGF (R&D Systems, UK, cat n°NFVEO) at 200μg/ml for NRP-1 / 4 μg/ml Heparin (Sigma, USA, cat n°H3393-10KU) and 100μg/ml for VEGF-R1 solution was added to each well; the plate was sealed and incubated at 37°C for 2 hours, shacking. The last incubation was carried out, after five washes with Buffer A, adding 100 μl/well Streptavidin-Horseradish Peroxidase (Amersham, Switzerland, cat n°: RPN4401) diluted 1:8000 with buffer A. After 1 hour shacking at 37°C under obscurity, the plate was washed 5 times with buffer A and 100 μl of SuperSignal west pico chemiluminescent substrate (Interchim, USA cat n°: 34078) were added. Luminescence was quantified with an EnVision™ 2101 Multilabel reader (Perkin Elmer, USA) and the data analyzed by Wallac Envision manager software. Signal is given as relative light units (RLU). Non-specific binding is defined as the signal measured in the absence of receptor coated on the microplate. The specific binding was calculated as the difference between total binding and non-specific binding. The percentage of inhibition was calculated as following:

$$\text{Inhibition \%} = 100 \times \frac{\text{(Signal measured – non specific binding signal)}}{\text{(Max binding signal obtained without inhibitor - non specific binding signal without receptor)}}$$

***Tube formation assay***

Collagen type I (1 mg/ml, BD) was mixed with 0.1N NaOH, 0.1% bicarbonate and 10X in medium EBM-2 and kept on ice until placed into the wells. The assay was prepared in 96-well plate format and the bottom layer of collagen was formed by adding 70 μL of the solution per well and allowing subsequent gelation at 37°C for 30 minutes. The HUVEC cells were then seeded on collagen at $8.10^5$ cells per well. After monolayer formation (one day), a second layer of collagen 200 μL per well was prepared according to the same protocol and gently placed on top of the cell layer. The compounds of the invention were added to the cells at the adhesion step (concentration corresponding to the Inhibitory Concentration 50 ($IC_{50}$)). After 24 and 48 hours of incubation at 37°C, tube formation was observed and pictures were taken.

***In vivo Studies***

Female (NOD-SCID) mice 5-6 weeks old were injected subcutaneously with MDA-MB-231 cells ($5.10^6$) at a single site on their right flanks. Tumors were allowed to grow (19 days) to less than 110 mm before being pair-matched by tumor size into treatment groups (8 mice/group). One group was treated with vehicles (8 mice/group receiving cremofor 12.5%/ethanol 12.5%/ H2O), one group was treated with 10 mg/kg of the compound **1** of the invention (8 mice/group receiving compound **1** + cremofor 12.5%/ethanol 12.5%/ $H_2O$), one group was treated with 50 mg/kg of the compound **1** of the invention (9 mice/group receiving compound **1** + cremofor 12.5%/ethanol 12.5%/ $H_2O$). All groups received daily by oral gavages for 5 consecutive days/weeks approximately 100 μL of a solution containing the compound **1** of the invention. Mice were weighed regularly to assess toxicity of the treatments and the tumors were measured with calipers (width x width x length x Pi/6) to determine tumor growth. Mice survival was assessed during the study and metastatic development was investigated in lung, spleen and liver.

**Results**

The presented results showed that the compounds **1, 2** and **3** of the invention were cytotoxic for endothelial cell lines (HUVEC cells) in culture. Moreover, these compounds also inhibited the adhesion of HUVEC cells (Figures 1A, B and C). It could therefore be hypothesized that the compounds of the invention presented an anti-angiogenic effect. To confirm this hypothesis, an in vitro experiment for assessing the formation of blood vessels by endothelial cells in culture was carried out. Figure 1D shows that the compounds **1** and **2** of the invention effectively inhibited the formation of new blood vessels.

Figures 1A, B and C also showed that the compounds **1, 2** and **3** of the invention are cytotoxic for cancer cells in vitro, and that these compounds inhibited the adhesion of cancer cells in culture. The cytotoxicity of the compounds **1** and **2** of the invention was confirmed for different cancer cell lines. The effect of the compounds was assessed using

wst-1 cytotoxic test (see for example Figure 2). In conclusion, the compounds **1** and **2** of the invention are cytotoxic for all the tested cancer cell lines, whatever their origin (breast, colon, pancreas, cervical, prostate or brain cancer or osteosarcoma), as shown in the Table 1 below.

| Cell lines | | Concentration | % of Inhibition | |
|---|---|---|---|---|
| Type | Name | | *Compound 1* | *Compound 2* |
| Breast Cancer | MDA-MB-438 | 1 μM | 55 % | 58 % |
| | | 0.5 μM | 40 % | 51 % |
| | | 0.1 μM | 4 % | 34 % |
| | | 0.05 μM | 2 % | 13 % |
| Colon Cancer | HCT-116 | 1 μM | 56 % | 60 % |
| | | 0.5 μM | 51 % | 58 % |
| | | 0.1 μM | 7 % | 49 % |
| | | 0.05 μM | 0 % | 25 % |
| Pancreas Cancer | PANC – 10.5 | 1 μM | 56 % | 65 % |
| | | 0.5 μM | 44 % | 62 % |
| | | 0.1 μM | 21 % | 51 % |
| | | 0.05 μM | 18 % | 35 % |
| Cervical Cancer | HeLa | 1 μM | 68 % | 67 % |
| | | 0.5 μM | 64 % | 64 % |
| | | 0.1 μM | 13 % | 62 % |
| | | 0.05 μM | 4 % | 49 % |
| Osteosarcoma | U2OS | 1 μM | 36 % | 44 % |
| | | 0.5 μM | 29 % | 41 % |
| | | 0.1 μM | 15 % | 31 % |
| | | 0.05 μM | 9 % | 18 % |
| Prostate Cancer | PC-3 | 1 μM | 47 % | 46 % |
| | | 0.5 μM | 25 % | 46 % |
| | | 0.1 μM | 3 % | 29 % |
| | | 0.05 μM | 0 % | 0 % |
| Brain Cancer | U87 glioblastoma | 1 μM | 54 % | 61 % |
| | | 0.5 μM | 45 % | 60 % |
| | | 0.1 μM | 0 % | 44 % |
| | | 0.05 μM | 0 % | 19 % |

Table 1

Taken together, these results indicated an effect of the compounds **1** and **2** of the invention against cancer cells in vitro.

Furthermore, the potential in vivo anti-cancer effect of these compounds was tested in mice. NOD-SCID mice were injected with tumor, and then fed with the compounds of the invention.

The results showed no significant change in body weight, demonstrating the absence of toxicity of the compound of the invention at a dose of 10 mg/kg as well as at a dose of 50 mg/kg (Figure 3A).

The compound **1** of the invention inhibited the development of the tumor. Indeed, it is clear on Figure 3B that the tumor size was smaller in mice fed with 10 mg/kg or 50 mg/kg of the compound **1.**

Moreover, the mice fed with the compound **1** of the invention presented a percentage of survival at day 35 of 100% for the 50 mg/kg dose and about 70% for the 10 mg/kg dose, compared to less that 50% for the control (Figure 3C).

Taken together, these results indicated an anti-cancer effect of the compound **1** of the invention in vivo.

The inventors showed that the compounds of the invention inhibited the binding of VEGF on its receptor Neuropilin-1 (NRP-1, see Table 2 below), and, to a lesser extent, the binding of VEGF on its receptor VEGF-R1 (see Table 3 below).

| Compound of the invention | % VEGF/NRP-1 binding inhibition |
|---|---|
| *Compound 1* | **35 ± 4** |
| *Compound 2* | **24 ± 2** |
| *Compound 3* | **46 ± 3** |

Table 2

| Compound of the invention | % VEGF/VEGF-R1 binding inhibition |
|---|---|
| *Compound 1* | **18 ± 3** |
| *Compound 2* | **7 ± 4** |
| *Compound 3* | **7 ± 3** |

Table 3

This result suggested that the compounds of the invention exerted their anti-angiogenic and anti-cancer effect through the inhibition of the pathway including VEGF, VEGF-R1 and NRP-1.

### Claims

1. A pharmaceutical composition comprising a compound of general formula (I),

(I)

wherein

- **Z1** is S or O, preferably S;
- -**Z2**- is -N**R$^a$**-, wherein **R$^a$** is H, OH, alkyl, alkoxy, aryle, alkenyl, alkynyl, amine, preferably R$^a$ is H; -N=; O; S; -C**R$^b$**=, wherein **R$^b$** is H, OH, alkyl, alkoxy, aryle, alkenyl, alkynyl, amine, $NO_2$, Cl, Br, I, F, preferably H; -CH**R$^c$**-, wherein **R$^c$** is H, OH, alkyl, alkoxy, aryle, alkenyl, alkynyl, amine, $NO_2$, Cl, Br, I, F, or -$CH_2$-; preferably -**Z2**- is -NH- or -N=;
- each of **R1** and **R2** is independently H, OH, alkyl, alkoxy, aryle, alkenyl, alkynyl, amine, $NO_2$, Cl, Br, I, F; preferably **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;

- **R5** is H, OH, $C_{1-4}$ alkyl, $C_{1-4}$ alkoxy, $C_{1-4}$ alkenyl, $C_{1-4}$ alkynyl, amine, preferably **R5** is $NH_2$, $NO_2$, Cl, F, Br, I; more preferably **R5** is H or $CH_3$; even more preferably **R5** is $CH_3$;
- each of **R8** and **R9** is independently, H, OH, alkyl, alkoxy, aryle, alkenyl, alkynyl, amine, $NO_2$, Cl, Br, I, F; preferably $R_8$ and $R_9$ together form a cycle comprising 5 or 6 atoms, preferably an heterocycle comprising 5 or 6 atoms, more preferably a 1,3-dioxacyclopentene or a 1,4-dioxane; and
- each of **R3, R4, R6, R7, R10** and **R11** is independently, H, OH, $C_{1-4}$ alkyl, $C_{1-4}$ alkoxy, $C_{1-4}$ alkenyl, $C_{1-4}$ alkynyl, amine, $NO_2$, F, Cl, Br, I; preferably H, $CH_3$, $OCH_3$, OH, $NH_2$, $NO_2$, Cl, F, Br, I; more preferably is H or $CH_3$; even more preferably is H,
- or esters or salts thereof; in association with at least one pharmaceutically acceptable vehicle.

2. The pharmaceutical composition according to Claim **1,** comprising the compound of general formula (I) wherein

- **Z1** is S or O, preferably S;
- **-Z2-** is -$NR^a$-, wherein $R^a$ is H, OH, alkyl, alkoxy, aryle, alkenyl, alkynyl, amine, preferably H; -N=; O; S; -$CR^b$=, wherein $R^b$ is H, OH, alkyl, alkoxy, aryle, alkenyl, alkynyl, amine, $NO_2$, Cl, Br, I, F, preferably H; -$CHR^c$-, wherein $R^c$ is H, OH, alkyl, alkoxy, aryle, alkenyl, alkynyl, amine, $NO_2$, Cl, Br, I, F, or -$CH_2$-, preferably -**Z2**- is -NH- or -N=;
- **R1** and **R2** together and with N and Z2 form an heterocycle eventually substituted, more preferably a substituted azocine, 3H-indole, indazole, 2-imidazoline, 2-pyrazoline, benzthiazole, purine, pyrimidine, pyridine, pyridazine, pyrazine, pyrazole, thiazole, isothiazole, oxazole, isoxazole, quinoline, isoquinoline, quinoxaline, quinazoline, 1-8-naphthyridine, perimidine, [1,10]-phenantroline, phthalazine, pteridine, triazole, triazine, furazan, 6H-1,2,5-thiadiazine, 1,3,4-thiadiazole, tetrazole, or a substituted imidazole and even more preferably a benzimidazole;
- **R5** is H or $C_{1-4}$alkyl;
- **R8** and **R9** together form a cycle comprising 5 or 6 atoms, preferably an heterocycle comprising 5 or 6 atoms, more preferably a 1,3-dioxacyclopentene or a 1,4-dioxane;
- **R3, R4, R6, R7, R10** and **R11** are H or $C_{1-4}$ alkyl.

3. The pharmaceutical composition according to Claim **1** or Claim **2,** comprising the compound of general formula (Ia):

R9 R8 H N O H N S N N H R5

(Ia)

wherein

- **R5** is H or CH3;
- **R8** and **R9** together form a 1,3-dioxacyclopentene or a 1,4-dioxane.

4. The pharmaceutical composition according to anyone of Claims **1** to **3,** comprising:

N-[5-(1H-benzimidazol-2-yl)-2-methylphenyl]-N'-(2,3-dihydro-1,4-benzodioxin -6- ylcarbonyl)thiourea

,

N-[3-(1H-benzimidazol-2-yl)phenyl]-N'-(2,3-dihydro-1,4-benzodioxin -6- ylcarbonyl)thiourea:

,

and/or
N-[3-(1H-benzimidazol-2-yl)phenyl]-N'-(1,3-benzodioxol-5-ylcarbonyl)thiourea

.

or their salts and esters, and mixtures thereof.

**5.** A medicament comprising a pharmaceutical composition according to anyone of Claims **1** to **4.**

**6.** A method for manufacturing a compound as described in anyone of Claims **1** to **4,** where a compound of general formula (II)

(II)

wherein **R1, R2, Z2, R3, R4, R5** and **R6** are as described in anyone of Claims **1** to **4** is reacted with a compound of general formula (IV)

(IV)

in a refluxing solvent, to give compounds of general formula (I).

7. The method according to Claim **6,** wherein the compound of general formula (IV) is obtained by reacting a compound of general formula (III)

(III)

with (i) SOCl2 and (ii) NH4**Z1**CN, wherein Z1 is as defined in claim **1,** in the presence of a base.

8. A pharmaceutical composition according to anyone of Claims **1** to **4,** for inhibiting the Neuropilin pathways.

9. A pharmaceutical composition comprising a therapeutically effective amount of a compound of general formula (I) in association with a pharmaceutically acceptable vehicle as described in anyone of Claims **1** to **4,** for use in prevention and/or treatment of a cancer in a subject in need thereof.

10. The pharmaceutical composition according to Claim **9** for use for inhibiting the growth of a tumor and/or for inhibiting the number and the development of cancerous cells within the body of the subject and/or for inhibiting the occurrence of metastases.

11. The pharmaceutical composition according to Claim **9** or Claim **10,** wherein the cancer is one of carcinoma, adenocarcinoma, lymphoma, blastoma, hepatoma, sarcoma; leukemia, such as, for example, squamous cell cancer; lung cancer, including small-cell lung cancer, non-small cell lung cancer, adenocarcinoma of the lung, and squamous carcinoma of the lung; cancer of the peritoneum; hepatocellular cancer; gastric or stomach cancer, including gastrointestinal cancer; pancreatic cancer, such as, for example, pancreatic carcinoma; glioblastoma; neuroblastoma; cervical cancer; ovarian cancer; liver cancer; bladder cancer; hepatoma; breast cancer; colon cancer, such as, for example, colon adenocarcinoma; colorectal cancer, such as, for example, colorectal carcinoma; endometrial or uterine carcinoma; salivary gland carcinoma; osteosarcoma; kidney or renal cancer; liver cancer; prostate cancer, such as, for example, prostate adenocarcinoma; vulval cancer; thyroid cancer; hepatic carcinoma and various types of head and neck cancer; B-cell lymphoma, including low grade/follicular non-Hodgkin's lymphoma (NHL), small lymphocytic (SL) NHL, intermediate grade/follicular NHL, intermediate grade diffuse NHL, high grade immunoblastic

NHL, high grade lymphoblastic NHL, high grade small non-cleaved cell NHL, bulky disease NHL, mantle cell lymphoma, AIDS-related lymphoma, and Waldenstrom's Macroglobulinemia, chronic lymphocytic leukemia (CLL), acute lymphoblastic leukemia (ALL), Hairy cell leukemia, chronic myeloblastic leukemia; and post-transplant lymphoproliferative disorder (PTLD), abnormal vascular proliferation associated with phakomatoses, edema, such as, for example that associated with brain tumors; and Meigs' syndrome.

**12.** The pharmaceutical composition according to anyone of Claims **9** to **11,** wherein said subject is diagnosed with a cancer and/or underwent a surgical operation to remove a tumor and/or was previously treated with an anti-cancer treatment, and/or is simultaneously treated with an anti-cancer treatment, and/or is planned to be treated with an anti-cancer treatment, wherein the anti-cancer treatment may be chemotherapy and/or radiotherapy.

**13.** A pharmaceutical composition comprising a therapeutically effective amount of a compound of general formula (I) in association with a pharmaceutically acceptable vehicle as described in anyone of Claims **1** to **4,** for use in prevention and/or treatment a disease, disorder or condition related to angiogenesis in a subject in need thereof.

**14.** A pharmaceutical composition according to claim **13,** for use for inhibiting the formation of neovessels and/or the adhesion of endothelial cells and/or the growth, development or division of endothelial cells.

**15.** The pharmaceutical composition according to claim **13** or Claim **14,** wherein the disease related to angiogenesis is an ophthalmic disease, a disease of the renal and/or urogenital tract; a disease of the digestive tract; a disease of the respiratory tract; a disease of bones or joints; a skin disease; an autoimmune disease; is consecutive to a graft; is a post-surgery reaction, a hyperproliferation or a hypertrophy; abnormal wound healing; chronic inflammation; a disease of the cardiovascular system or of blood or an infection.

**A-** ***Compound 1***

**HUVEC CELLS**

100
75
50
25
0
Adhesion inhibition (%)
$IC_{50}$ = 0,16 µM
±0,01
n = 6
-10 -9 -8 -7 -6 -5 -4 -3 -2 -1
Log(c)
100
75
50
25
0
Cytotoxicity inhibition (%)
$IC_{50}$ = 0,21 µM
±0,03
n = 5
-10 -9 -8 -7 -6 -5 -4 -3 -2 -1
Log(c)

**MDA-MB-231CELLS**

100
75
50
25
0
Adhesion inhibition (%)
$IC_{50}$ = 0,6 µM
±0,07
n = 6
-10 -9 -8 -7 -6 -5 -4 -3 -2 -1
Log(c)
100
75
50
25
0
Cytotoxicity inhibition (%)
$IC_{50}$ = 0,7 µM
±0,37
n = 3
-10 -9 -8 -7 -6 -5 -4 -3 -2 -1
Log(c)

FIG. 1A

**B-** ***Compound 2***

**HUVEC CELLS**

Adhesion inhibition (%)
100
75
50
25
0
-10 -9 -8 -7 -6 -5 -4 -3 -2 -1
Log(c)
IC50 = < 0,1 µM
± 0
n = 2
Cytotoxicity inhibition (%)
100
75
50
25
0
-10 -9 -8 -7 -6 -5 -4 -3 -2 -1
Log(c)
IC50 = < 0,1 µM
± 0
n = 2

**MDA-MB-231CELLS**

Adhesion inhibition (%)
100
75
50
25
0
-10 -9 -8 -7 -6 -5 -4 -3 -2 -1
Log(c)
IC50 = 0,5 µM
± 0
n = 2
Cytotoxicity inhibition (%)
100
75
50
25
0
-10 -9 -8 -7 -6 -5 -4 -3 -2 -1
Log(c)
IC50 = 0,6 µM
± 0
n = 2

FIG. 1B

**C-** ***Compound 3***

**HUVEC CELLS**

Adhesion inhibition (%)
100
75
50
25
0
-10 -9 -8 -7 -6 -5 -4 -3 -2 -1
Log(c)
$IC_{50}$ = 0,3 µM
± 0,01
n = 2
Cytotoxicity inhibition (%)
100
75
50
25
0
-10 -9 -8 -7 -6 -5 -4 -3 -2 -1
Log(c)
$IC_{50}$ = 0,3 µM ± 0
n = 2

**MDA-MB-231CELLS**

Adhesion inhibition (%)
100
75
50
25
0
-10 -9 -8 -7 -6 -5 -4 -3 -2 -1
Log(c)
$IC_{50}$ = 6 µM ± 0
n = 2
Cytotoxicity inhibition (%)
100
75
50
25
0
-10 -9 -8 -7 -6 -5 -4 -3 -2 -1
Log(c)
$IC_{50}$ = 10 µM ± 0
n = 2

**D**

*Control*
*Compound 1*
*Compound 2*

**FIG. 1C and 1D**

compound 1
compound 2
Drug cytotoxicity (%)
100
75
50
25
0
68%
64%
67%
64%
62%
49%
1
0,5
0,1
0,05
(µM)

FIG. 2

A-

Boby weight
Days
Control
10 mg/kg
50 mg/kg

B-

Tumour size (mm³)
Days
control n=7
10 (mg) n=8
50 mg n=7

FIG. 3 A-B

C

150
100
50
0
Percent Survival
10
20
30
40
Days
Control n=7
10 mg/kg n=6
50 mg/kg n=7

FIG. 3 C

Europäisches Patentamt
European Patent Office
Office européen des brevets

# EUROPEAN SEARCH REPORT

Application Number
EP 11 16 6117

**DOCUMENTS CONSIDERED TO BE RELEVANT**

| Category | Citation of document with indication, where appropriate, of relevant passages | Relevant to claim | CLASSIFICATION OF THE APPLICATION (IPC) |
|---|---|---|---|
| X | WO 2011/010013 A1 (CENTRE NAT RECH SCIENT [FR]; RUAT MARTIAL [FR]; FAURE HELENE [FR]; TRA) 27 January 2011 (2011-01-27)<br>* page 29; compound XII *<br>----- | 1,5,8 | INV.<br>A61K31/17<br>A61P35/00 |
| A | EP 1 870 414 A1 (KIRIN PHARMA KK [JP]) 26 December 2007 (2007-12-26)<br>* page 27; compound 23 *<br>----- | 1-5,8-15 | |

TECHNICAL FIELDS SEARCHED (IPC)
A61K

~~The present search report has been drawn up for all claims~~

| Place of search | Date of completion of the search | Examiner |
|---|---|---|
| Munich | 16 September 2011 | Uryga-Polowy, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches
Patentamt
European
Patent Office
Office européen
des brevets

Application Number

EP 11 16 6117

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-5, 8-15

- [ ] The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number
EP 11 16 6117

The Search Division considers that the present European patent application does not comply with the requirements of unity of invention and relates to several inventions or groups of inventions, namely:

1. claims: 1-5, 8-15

   a pharmaceutical composition comprising a compound of formula (I) for use in the treatment of neuropilin related disorders

   ---

2. claims: 6, 7

   a method for manufacturing a compound of formula (I) as described in claims 1-4

   ---

## ANNEX TO THE EUROPEAN SEARCH REPORT ON EUROPEAN PATENT APPLICATION NO. EP 11 16 6117

This annex lists the patent family members relating to the patent documents cited in the above-mentioned European search report. The members are as contained in the European Patent Office EDP file on
The European Patent Office is in no way liable for these particulars which are merely given for the purpose of information.

16-09-2011

| Patent document cited in search report | Publication date | Patent family member(s) | Publication date |
|---|---|---|---|
| WO 2011010013 A1 | 27-01-2011 | FR 2948367 A1 | 28-01-2011 |
| | | WO 2011010013 A1 | 27-01-2011 |
| EP 1870414 A1 | 26-12-2007 | AU 2006229343 A1 | 05-10-2006 |
| | | CA 2603125 A1 | 05-10-2006 |
| | | CN 101189241 A | 28-05-2008 |
| | | EP 1870414 A1 | 26-12-2007 |
| | | KR 20080007443 A | 21-01-2008 |
| | | US 2009270391 A1 | 29-10-2009 |
| | | WO 2006104161 A1 | 05-10-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 03035100 A
- WO 2007056470 A
- US 7335357 B
- WO 2009099959 A
- WO 9955855 A
- WO 2009130422 A

### Non-patent literature cited in the description


- **ELLIS LM.** *Mol Cancer Ther,* 2006, vol. 5 (5), 1099-107
- **JARVIS et al.** *J. Med. Chem.,* 2010, vol. 53, 2215-26